# EUROPÄISCHE PATENTANMELDUNG

(11) **EP 4 257 579 A1**
(43) Veröffentlichungstag der Anmeldung: **11.10.2023**
(21) Anmeldenummer: 22020154.5
(22) Anmeldetag: 07.04.2022
(51) Int. Cl.: C07C 41/01, C07C 43/04

(54) **VERFAHREN UND ANLAGE ZUR HERSTELLUNG VON DIMETHYLETHER**

(71) Anmelder: Linde GmbH, 82049 Pullach (DE)
(72) Erfinder: Winkler, Florian, 82049 Pullach (DE); Behrens, Axel, 82049 Pullach (DE); Peschel, Andreas, 82049 Pullach (DE); Kiendl, Isabel, 82049 Pullach (DE)
(74) Vertreter: Fischer, Werner

(57) **Zusammenfassung**

Ein Verfahren (100) zur Herstellung von Dimethylether wird vorgeschlagen, bei dem ein die Reaktanden Kohlenmonoxid und/oder Kohlendioxid und Wasserstoff enthaltendes Synthesegas in einer Strömungsrichtung unter teilweiser Umsetzung der Reaktanden und Bildung von Methanol an einem oder mehreren Methanolkatalysatoren und Umsetzung zumindest eines Teils des Methanols an einem oder mehreren Dimethyletherkatalysatoren durch einen Synthesereaktor (10) geführt wird, in dem der Methanolkatalysator und der Dimethyletherkatalysator in einer gemeinsamen Katalysezone (14) bereitgestellt sind. Es ist vorgesehen, dass die Katalysezone (14) zumindest zwei Teilzonen (A, B, C) aufweist, die in der Strömungsrichtung hintereinander angeordnet sind, wobei die Teilzonen (A, B, C) den oder die Methanolkatalysatoren in ansteigenden Aktivitäten aufweisen. Eine entsprechende Anlage ist ebenfalls Gegenstand der vorliegenden Erfindung.

## Beschreibung

Die Erfindung betrifft ein Verfahren und eine Anlage zur Herstellung von Dimethylether.

### Hintergrund

Bei Dimethylether (DME) handelt es sich um den strukturell einfachsten Ether. Dimethylether enthält zwei Methylgruppen als organische Reste. Dimethylether ist polar und findet herkömmlicherweise in flüssiger Form als Lösungsmittel Verwendung. Dimethylether kann ferner als Kühlmittel eingesetzt werden und herkömmliche Fluorchlorkohlenwasserstoffe ersetzen. Neuerdings wird Dimethylether zunehmend als Ersatz für Brenngas (Flüssiggas) und Kraftstoffe wie Diesel eingesetzt.

Dimethylether kann entweder direkt aus Methanol oder indirekt aus Erd- oder Biogas erzeugt werden. Im letzteren Fall wird das Erd- oder Biogas zunächst zu Synthesegas umgesetzt. Das Synthesegas wird anschließend entweder in einer zweistufigen Reaktion zu Methanol und anschließend zu Dimethylether oder in einer einstufigen Reaktion direkt zu Dimethylether konvertiert.

Unter Synthesegas soll hier ein Gasgemisch verstanden werden, das zumindest Kohlenmonoxid und Wasserstoff in wechselnden Anteilen, die zusammen jedoch den überwiegenden Teil des Gasgemischs ausmachen, enthält. Auch Kohlendioxid kann enthalten sein, welches durch eine reverse Wassergasshiftreaktion (RWGS) zu weiterem Kohlenmonoxid umgesetzt werden kann.

Synthesegas kann durch Pyrolyse von Kohle, Öl, kohlenstoffhaltigen Abfällen, Biomasse oder anderen Ausgangsstoffen, durch Trockenreformierung (Dry Reforming) von Erdgas mit Kohlendioxid, durch Dampfreformierung (Steam Reforming) von Erdgas, durch autotherme Reformierung (ATR) von Erdgas, durch partielle Oxidation (POX) von Kohlenwasserstoffen, insbesondere Erdgas bzw. Methan, oder Kombinationen aus den genannten Verfahren, gewonnen werden.

Die Synthese von Dimethylether aus Synthesegas ist thermodynamisch und wirtschaftlich vorteilhaft gegenüber einer Synthese aus Methanol.

Die vorliegende Erfindung betrifft insbesondere die einstufige oder direkte Synthese von Dimethylether, worunter eine Synthese verstanden wird, bei der sämtliche Reaktionen in einunddemselben Reaktor und in einunddemselben Katalysatorbett ablaufen. Bei den ablaufenden Reaktionen handelt es sich um eine Reaktion von Wasserstoff mit Kohlenmonoxid und/oder Kohlendioxid zu Methanol und eine (Weiter-) Reaktion von Methanol zu Dimethylether und Wasser. Die einstufige Synthese von Dimethylether ist beispielsweise aus der US 4,536,485 A und der US 5,189,203 A bekannt. Herkömmlicherweise werden hierbei Hybridkatalysatoren verwendet. Die Reaktion ist exotherm und erfolgt typischerweise bei einer Temperatur von 200 bis 300 °C und bei einem Druck von 20 bis 100 bar.

Zur einstufigen Synthese von Dimethylether werden normalerweise aufrechtstehende Rohrreaktoren eingesetzt, die von unten mit druckbeaufschlagtem und erhitztem Synthesegas beschickt werden. Ein in dem Rohrreaktor erhaltener Produktstrom wird kopfseitig entnommen, gekühlt und einer Trennung zugeführt.

Der Produktstrom enthält typischerweise neben Dimethylether nicht umgesetzte Komponenten des Synthesegases sowie weitere Reaktionsprodukte. Typischerweise weist der Produktstrom neben Dimethylether zumindest Methanol, Wasser, Kohlendioxid, Kohlenmonoxid und Wasserstoff sowie in geringerer Menge Methan, Ethan, organische Säuren und höhere Alkohole auf.

In einem Gasgemisch, das aus dem Produktstrom gebildet wird, sind damit typischerweise neben Dimethylether noch Kohlendioxid und leichter als Kohlendioxid siedende Komponenten wie Wasserstoff und Kohlenmonoxid enthalten. Je nach Produktspezifikation müssen diese zumindest teilweise abgetrennt werden.

Die Zusammensetzung des Produktstroms (dieser oder ein hieraus gebildetes Gasgemisch wird nachfolgend auch als Dimethylether enthaltendes Produktgemisch bezeichnet) wird dabei insbesondere durch die gewählten Reaktionsbedingungen während der Synthese bestimmt. Je näher diese Zusammensetzung stromab des Reaktors an der geforderten Spezifikation liegt, desto geringer ist der Aufwand, der zur Aufreinigung des Produktstroms betrieben werden muss.

Insbesondere in Bezug auf die hier verwendete Terminologie, aber auch auf grundsätzliche Überlegungen die (insbesondere direkte) Synthese von Dimethylether aus Synthesegas betreffend, sei auf den Artikel von Kiendl et al., Chem. Ing. Tech. 2020, 92, No. 6, 736-745, verwiesen.

In der Praxis weisen sowohl bekannte zweistufige Reaktionsverfahren zur Herstellung von Dimethylether als auch einstufige Verfahren gewisse Nachteile auf, die die vorliegende Erfindung überwinden will.

### Offenbarung der Erfindung

Diese Aufgabe wird durch ein Verfahren und eine Anlage mit den Merkmalen der jeweiligen unabhängigen Patentansprüche gelöst. Vorteilhafte Ausgestaltungen der sind Gegenstand der Unteransprüche sowie der nachfolgenden Beschreibung.

Bei der zweistufigen Dimethylethersynthese wird, wie erwähnt, in einem ersten Schritt Methanol hergestellt, welches in einem zweiten Schritt zu Dimethylether konvertiert wird. Beide Reaktionen werden separat durchgeführt und sind gleichgewichtslimitiert. Der Vorteil der Kombination beider Reaktionen zur Realisierung höherer Umsätze wird bei zweistufigen Verfahren nicht genutzt.

Direktsynthesesysteme bestehen aus einer Mischung beider Katalysatoren oder aus einem bifunktionellen Katalysatorsystem. Nachteile einer solchen Systems sind nachfolgend erläutert. Derzeit werden in Systemen mit zwei unterschiedlichen Katalysatoren Methanolkatalysatoren und Dimethyletherkatalysatoren in unterschiedlichen Verhältnissen gemischt, um unterschiedliche Aktivitäten zu erreichen. Das hat aber immer die Folge, dass bei hohen Anteilen des Methanolkatalysators der Anteil des Dimethyletherkatalysators sinkt. Dies führt zur geringeren Umsetzung des gebildeten Methanols und somit zu einem niedrigeren Kohlenmonoxidumsatz. Vergrößert man den der Anteil des Dimethyletherkatalysators, so wird der Anteil des Methanolkatalysators reduziert, was wiederum zu geringeren Umsätzen der Kohlenstoffoxide insgesamt führt.

Vorliegend wird unter einer Methanolreaktion bzw. einem Methanolkatalysator die Reaktion zu Methanol bzw. der dabei verwendete Katalysator bzw. unter einer Dimethyletherreaktion bzw. einem Dimethyletherkatalysator die Reaktion zu Dimethylether bzw. der dabei verwendete Katalysator verstanden.

Die bei der Herstellung von Dimethylether ablaufenden Reaktionen sind stark exotherm. Sie sind prinzipiell gleichgewichtslimitiert, jedoch wird durch die Kombination der Methanolsynthese mit der Folgereaktion zu Dimethylether das Gleichgewicht der Gesamtreaktion im Gegensatz der reinen Methanolsynthese hin zu hohen gewünschten Kohlenstoffoxidkonversionen verschoben, was die Exothermie der Reaktion, vor allem zu Beginn begünstigt.

Die Temperatur des Reaktionsgases darf bestimmte Werte nicht überschreiten sowie unterschreiten. In beiden Fällen kommt es zu beschleunigten Alterungsprozessen. In Kombination mit Wasser führen niedrige Temperaturen zu einer weiteren Beschleunigung der Alterung. Ein optimaler Betriebstemperaturbereich kann beispielsweise bei 240 bis 260 °C liegen. Die Temperaturen sollten dabei 280 °C nicht überschreiten und 220 °C nicht unterschreiten.

In der Regel verwendet man zur Kontrolle der Reaktion gekühlte Reaktorkonzepte. Um Temperaturspitzen am Reaktoreingang zu kontrollieren, benötigt man in diesem Bereich niedrige Temperaturen des Kühlmittels. Dies hat zur Folge, dass gegen Ende der Reaktorlänge niedrige Temperaturen vorliegen und die Reaktionsraten sowohl der Dimethyletherreaktion als auch der Methanolreaktion sinken und das Gleichgewicht nicht oder sehr schwer erreicht werden. Alternativ ist ein mit hohem apparativen Aufwand verbundenes zweites Kühlsystem erforderlich, um die Reaktion kinetisch wieder zu beschleunigen.

Optional oder zusätzlich zur Kühlmitteltemperatur dienen kleine Rohrdurchmesser des Rohrbündelreaktors zur besseren Energieabfuhr. Dies hat jedoch zur Folge, dass die Anzahl der Rohre in einem Reaktor steigt und somit Baubarkeitsgrenzen kommerzieller Größenordnungen schnell erreicht werden und der apparative Aufwand steigt. Dehydratisierungskatalysatoren (im Speziellen sehr aktive Zeolite und Sirale) neigen zu Rußbildung und Nebenproduktbildung in der DME-Synthese beim Überschreiten von zu hohen Temperaturen (über 290 °C). Diese Temperaturen können durch Temperaturspitzen im Reaktor überschritten werden.

Die vorliegende Erfindung schlägt ein Verfahren zur Herstellung von Dimethylether vor, bei dem ein die Reaktanden Kohlenmonoxid und/oder Kohlendioxid und Wasserstoff enthaltendes Synthesegas in einer Strömungsrichtung unter teilweiser Umsetzung der Reaktanden und Bildung von Methanol an einem oder mehreren Methanolkatalysatoren und Umsetzung zumindest eines Teils des Methanols an einem oder mehreren Dimethyletherkatalysatoren durch einen Synthesereaktor geführt wird, in dem der Methanolkatalysator und der Dimethyletherkatalysator in einer gemeinsamen Katalysezone bereitgestellt sind. Die vorliegende Erfindung betrifft also die einstufige oder direkte Dimethylethersynthese. Hierbei ist vorgesehen, dass die Katalysezone zumindest zwei Teilzonen aufweist, die in der Strömungsrichtung hintereinander angeordnet sind, wobei die Teilzonen den oder die Methanolkatalysatoren in ansteigenden Aktivitäten aufweisen. Die Aktivität des oder der Methanolkatalysatoren wird dabei unter anderem durch die Bedingungen, denen dieser oder diese Katalysatoren ausgesetzt sind, aber insbesondere auch durch die Eigenschaften des oder der Katalysatoren beeinflusst, die der Fachmann in geeigneter Weise auswählen und damit die Aktivität einstellen kann. Es handelt sich z.B. um unterschiedliche Zusammensetzungen (Aktiv-Komponenten gegenüber Trägermaterial oder Hilfsstoffen), unterschiedliche aktive Oberflächen (beeinflusst durch die (äußere) Oberfläche und die vorhandenen Poren) sowie um eine unterschiedliche Anzahl und Größe von Aktivzentren.

Gemäß einem Aspekt der Erfindung wird, anders ausgedrückt, ein "Layering" der Reaktorschüttung in spezifischer Weise gestaltet, wodurch vorteilhafterweise die Temperaturkontrolle in einem optimalen Betriebsfenster gehalten werden kann, ohne den Anteil an dem Dimethyletherkatalysator zu reduzieren, der der für das Zielprodukt notwendig ist. Hierdurch wird eine Temperaturkontrolle ohne Konversionsverluste gewährleistet.

Unter Layering wird das Verwenden eines oder mehrerer unterschiedlich aktiver Methanolkatalysatoren in der (physikalischen) Katalysatormischung verstanden. Es werden insbesondere mindestens zwei und maximal drei verschiedene aktive Zonen, hier auch als "Teilzonen" bezeichnet, verwendet. Die Aktivität des oder der Methanolkatalysatoren ist aufsteigend. Dies hat den Vorteil, dass die höchste Katalysatoraktivität bei der niedrigsten Temperatur im Reaktor vorliegt und somit die Methanolgleichgewichtsreaktion gefördert wird und die niedrigste Katalysatoraktivität des oder der Methanolkatalysatoren am Reaktoranfang vorliegt, an dem die höchste Exothermie erwartet wird. Man erreicht dadurch eine Temperatur- und Reaktionskontrolle, ohne zwingend den Anteil des oder der Dimethyletherkatalysatoren lokal zu reduzieren oder lokal zu erhöhen, auch wenn diese im Rahmen der vorliegenden Erfindung nicht ausgeschlossen ist.

Die vorliegende Erfindung schafft insbesondere ein effizientes Verfahren zur direkten Synthese von Dimethylether mit einer verbesserten bzw. einfacheren Temperaturkontrolle des Synthesereaktors und einer Verbesserung der Katalysatorstabilität (Langlebigkeit) durch Vermeidung von zu hohen und zu niedrigen Temperaturen. Durch die örtliche Temperaturkontrolle kann die Wärmeabfuhr zu Gunsten größerer Rohrradien reduziert werden. Es ist insgesamt eine Reduzierung des apparativen Aufwandes möglich. Vor allem können zu niedrige Kühlmitteltemperaturen vermieden werden und es ist insbesondere kein zweites Kühlsystem für höhere Temperaturen am Reaktoraustritt erforderlich. Durch eine mögliche Wahl eines engeren Fensters der Reaktionsparameter verringern sich die Anforderungen an mögliche Katalysatoren (Methanol- und Dimethyletherkatalysatoren) durch die Kontrolle des Parameters Temperatur.

Der Methanolkatalysator oder zumindest einer der mehreren Methanolkatalysatoren kann insbesondere Kupfer aufweisen und/oder der Dimethyletherkatalysator oder zumindest einer der mehreren Dimethyletherkatalysatoren kann insbesondere eine saure Komponente aufweisen. Gemäß einer Ausgestaltung der Erfindung kann der Methanolkatalysator oder zumindest einer der mehreren Methanolkatalysatoren die Komponenten Kupfer, Zinkoxid und Dialuminiumtrioxid aufweisen und/oder der Dimethyletherkatalysator oder zumindest einer der mehreren Dimethyletherkatalysatoren kann ein oder mehrere Gamma-Aluminiumoxide, Sirale und/oder Zeolithe aufweisen. Die vorliegende Erfindung entfaltet ihre Vorteile insbesondere bei derartigen Katalysatorsystemen.

Eine Ausgestaltung der Erfindung kann auch vorsehen, dass in den Teilzonen mehrere unterschiedliche Methanolkatalysatoren und/oder mehrere unterschiedliche Dimethyletherkatalysatoren mit unterschiedlicher Wasser- und/oder Kohlendioxidverträglichkeit verwendet werden.

Im Rahmen der vorliegenden Erfindung kann neben des zuvor erwähnten Layerings zur Temperaturkontrolle also ein (weiteres) Layering auch bewusst zur robusteren Performance des Reaktorsystems eingesetzt werden. Da im Synthesereaktor, wegen der Methanolbildung und der parallel laufenden Bildung von Dimethylether sowie der parallelen Shiftreaktion von Kohlenmonoxid zu Kohlendioxid, trockene bis hin zu kohlendioxidreichen und gleichzeitig hydrothermal stark belastete Bereiche existieren, ist eine Verwendung unterschiedlicher Katalysatoren hinsichtlich ihrer Bildung von und/oder Beständigkeit gegenüber Wasser und/oder hinsichtlich ihrer Bildung von und/oder Beständigkeit gegenüber Kohlendioxid besonders vorteilhaft. Somit ist ein Layering auch hin sichtlich der besseren Haltbarkeit des Katalysators durch Einsatz unterschiedlicher Katalysatoren mit verschiedener Verträglichkeiten (ggf. im Kompromiss mit deren Umsetzung) realisierbar.

Gemäß Ausgestaltungen der vorliegenden Erfindung können in den Teilzonen jeweils eine physikalische Mischung des einen oder der mehreren Methanolkatalysatoren und des einen oder der mehreren Dimethyletherkatalysatoren eingebracht sein. Die physikalische Mischung kann insbesondere jeweils 40 bis 80 oder 50 bis 75 Gewichtsanteile des einen oder der mehreren Methanolkatalysatoren und in einem verbleibenden Gewichtsanteil den einen oder der mehreren Dimethyletherkatalysatoren aufweisen. Der Gehalt bzw. Gewichtsanteile des oder der Dimethyletherkatalysatoren muss also nicht aus den erläuterten Gründen reduziert werden, was eine besonders gute Umsetzung ermöglicht. Die Gehalte des einen oder der mehreren Methanolkatalysatoren und/oder des einen oder der mehreren Dimethyletherkatalysatoren sind insbesondere in den Katalysezonen im Wesentlichen unverändert bzw. weichen um nicht mehr als 10 Prozent oder im Rahmen von Fertigungstoleranzen voneinander ab.

In dem vorgeschlagenen Verfahren wird insbesondere ein Rohrreaktor bzw. Rohrbündelreaktor bekannter Art verwendet, in dem Reaktionsrohre angeordnet sind, die zumindest zum Teil von einem Kühlmedium umströmt werden. Die Reaktionsrohre können insbesondere in Vielzahl (beispielsweise in einer Anzahl von 1.000 bis 40.000) in einem Druckbehälter parallel oder im Wesentlichen parallel durch eine Kühlzone geführt sein und in dieser mit dem Kühlmedium eines oder mehrerer, insbesondere aber genau eines, Kühlmedienkreislaufs in thermischen Kontakt gebracht werden. Das Kühlmedium kann dabei im Gleich- oder Gegenstrom mit bzw. zu dem Synthesegas geführt werden.

In Ausgestaltungen der vorliegenden Erfindung kann das Kühlmedium aus einem oder mehreren Thermoölen, Siedewasser und einer oder mehreren Salzschmelzen oder Kombinationen hiervon ausgewählt sein. Eine Temperatur des Kühlmediums kann dabei insbesondere in einem Bereich von 200 bis 300 oder 230 bis 280 °C liegen. Mit anderen Worten ermöglicht es die vorliegende Erfindung also, die Temperatur des Kühlmediums nur gering abzusenken.

Gemäß Ausgestaltungen der vorliegenden Erfindung kann zumindest ein Teil der Reaktionsrohre einen Innendurchmesser von 20 bis 60 oder 24 bis 50 mm aufweisen. Durch die verbesserte Temperaturkontrolle können also im Rahmen der vorliegenden Erfindung vergleichsweise weitlumige Reaktionsrohre verwendet werden.

In einer Ausgestaltung des erfindungsgemäß vorgeschlagenen Verfahrens kann ein gesamtes katalytisches Volumen, das innerhalb der Reaktionsrohre angeordnet ist, mit einer stündlichen Gasraumgeschwindigkeit von 1.500 bis 5.000 oder 2.000 bis 4.000 pro Stunde durchströmt werden. Auf diese Weise kann der Katalysator möglichst lange im optimalen Bereich (nahe am Gleichgewichtsumsatz) betrieben werden, was in dem genannten Bereich der Gasraumgeschwindigkeiten der Fall ist. Die Erfindung ermöglicht dabei eine vorteilhafte Temperaturkontrolle.

In einer Ausgestaltung der vorliegenden Erfindung kann das Synthesegas auf einer Temperatur in einem Bereich von 200 bis 300, 230 bis 280 oder 240 bis 275 °C durch den Synthesereaktor geführt werden. Die vorliegende Erfindung ermöglicht dabei die Verwendung relativ enger Temperaturbereiche, ohne eine Schädigung des oder der jeweiligen Katalysatoren befürchten zu müssen.

Eine Anlage zur Herstellung von Dimethylether, die einen Synthesereaktor aufweist, ist ebenfalls Gegenstand der vorliegenden Erfindung. Die Anlage weist Mittel auf, die dafür eingerichtet sind, ein die Reaktanden Kohlenmonoxid und/oder Kohlendioxid und Wasserstoff enthaltendes Synthesegas in einer Strömungsrichtung unter teilweiser Umsetzung der Reaktanden und Bildung von Methanol an einem oder mehreren Methanolkatalysatoren und Umsetzung zumindest eines Teils des Methanols an einem oder mehreren Dimethyletherkatalysatoren durch den Synthesereaktor zu führen, wobei in dem Synthesereaktor der Methanolkatalysator und der Dimethyletherkatalysator in einer gemeinsamen Katalysezone bereitgestellt sind, dadurch gekennzeichnet, dass die Katalysezone zumindest zwei Teilzonen aufweist, die in der Strömungsrichtung hintereinander angeordnet sind, wobei die Teilzonen den oder die Methanolkatalysatoren in ansteigenden Aktivitäten aufweisen.

Zu Merkmalen und Vorteilen einer entsprechenden Anlage und möglicher Ausgestaltungen sei auf die obigen Erläuterungen bezüglich des erfindungsgemäßen Verfahrens und seiner Ausgestaltungen ausdrücklich verwiesen.

Anlagen gemäß Ausgestaltungen der vorliegenden Erfindung weisen insbesondere Mittel auf, die sie zur Durchführung eines vorstehend beschriebenen Verfahrens und entsprechender Ausgestaltungen ertüchtigen. Die erfindungsgemäße Anlage bzw. deren vorteilhafte Ausgestaltungen profitieren dementsprechend von den Vorteilen des jeweils entsprechenden Verfahrens in analoger Weise und umgekehrt.

Im Folgenden werden weitere Merkmale und Vorteile der vorliegenden Erfindung anhand von Ausführungsbeispielen unter Bezugnahme auf die beiliegende Zeichnung näher erläutert.

### Kurze Beschreibung der Figuren

Figur 1 zeigt eine vorteilhafte Ausgestaltung eines Synthesereaktors zum Einsatz in einem Verfahren gemäß einer Ausgestaltung der Erfindung.
Figur 2 zeigt ein Temperaturprofil bei Verwendung eines Synthesereaktors bzw. Verfahrens gemäß einer nicht erfindungsgemäßen Ausgestaltung.
Figur 3 zeigt ein Temperaturprofil bei Verwendung eines Synthesereaktors bzw. Verfahrens gemäß einem nicht erfindungsgemäßen Vergleichsbeispiel.
Figur 4 zeigt ein Temperaturprofil bei Verwendung eines Synthesereaktors bzw. Verfahrens gemäß einer Ausgestaltung der Erfindung.

### Ausführungsform(en) der Erfindung

In den Figuren werden Verfahrensschritte und Vorrichtungskomponenten teilweise gemeinsam beschrieben bzw. mit identischen Bezugszeichen angegeben. Generell betreffen Erläuterungen zu Verfahrensschritten die entsprechenden Vorrichtungskomponenten in gleicher Weise und umgekehrt.

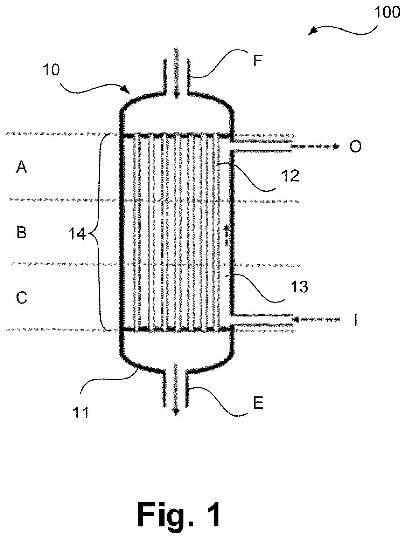

In Figur 1 ist ein eine vorteilhafte Ausgestaltung eines Synthesereaktors 10 zum Einsatz in einem Verfahren 100 gemäß einer Ausgestaltung der Erfindung veranschaulicht. Der Synthesereaktor 10 weist einen Reaktorbehälter 11 auf, innerhalb dessen Reaktionsrohre 12 angeordnet sind, welche parallel durch eine Kühlzone 13 verlaufen. Der Synthesereaktor 10 weist im dargestellten Beispiel Mittel auf, um über einen Einlass F den Reaktionsrohren 12 ein die Reaktanden Kohlenmonoxid und/oder Kohlendioxid und Wasserstoff enthaltendes Synthesegas zuzuführen und das Synthesegas in einer hier jeweils durch durchgezogene Pfeile angedeutete Strömungsrichtung unter teilweiser Umsetzung der Reaktanden und Bildung von Methanol an einem oder mehreren Methanolkatalysatoren und Umsetzung zumindest eines Teils des Methanols an einem oder mehreren Dimethyletherkatalysatoren durch den Synthesereaktor 10 bzw. dessen Reaktionsrohre 12 zu führen. Über einen Auslass E kann dem Synthesereaktor 10 dabei ein Effluent bzw. (Roh-)Produktgas entnommen und in jeder beliebigen Weise weiterverarbeitet werden.

Der Methanolkatalysator und der Dimethyletherkatalysator sind in einer gemeinsamen Katalysezone 14 bereitgestellt, wobei in der hier veranschaulichten Ausgestaltung der Erfindung die Katalysezone 14 drei (mit gestrichelten Horizontallinien angedeutete) Teilzonen A, B, C aufweist, die in der Strömungsrichtung hintereinander angeordnet ist, wobei die Teilzonen A, B, C den oder die Methanolkatalysatoren, wie zuvor ausführlich erläutert, in ansteigenden Aktivitäten aufweisen.

Die Kühlung der Reaktionsrohre 12 wird im hier veranschaulichten Beispiel durch (genau) einen Kühlmedienkreislauf bewirkt, wie durch gestrichelte Pfeile veranschaulicht. Ein entsprechendes Kühlmedium wird dabei über einen Einlass I in die Kühlzone 13 eingespeist, im Gegenstrom zu dem Synthesegas in den Reaktionsrohren 12 geführt, und über einen Auslass O entnommen.

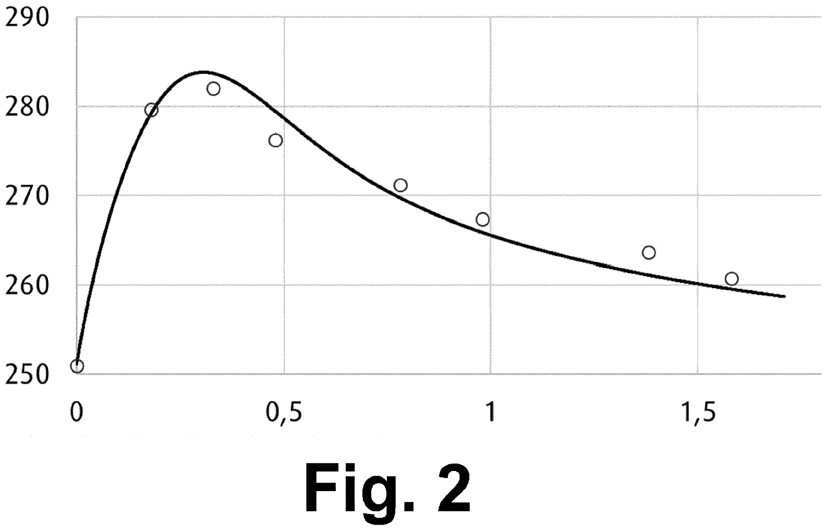

Figur 2 zeigt ein Temperaturprofil bei Verwendung eines Synthesereaktors bzw. Verfahrens gemäß einer nicht erfindungsgemäßen Ausgestaltung. Hier und in den nachfolgenden Profilen sind jeweils eine Reaktor- bzw. Katalysatorschüttungslänge in Metern auf der Horizontalachse gegenüber einer Temperatur in °C auf der Vertikalachse aufgetragen. Mit Kreisen sind gemessene Temperaturwerte, mit einer durchgezogenen Linie simulierte Temperaturwerte veranschaulicht.

In dem zugrundeliegenden Synthesereaktor wurde ein nicht erfindungsgemäßes, einfach strukturiertes Katalysatorbett verwendet. Eine stündliche Gasraumgeschwindigkeit, bezogen auf das gesamte Katalysatorvolumen, betrug 3000 pro Stunde. Ferner wurden ein Druck von 60 bar (abs.), eine Kühlmitteltemperatur von 250 °C sowie ein Wasserstoffgehalt von 50 Volumenprozent, ein Kohlendioxidgehalt von 25 Volumenprozent, ein Kohlendioxidgehalt von 5 Volumenprozent und ein Stickstoffgehalt von 20 Volumenprozent im eingesetzten Synthesegas verwendet. Eine gemessene Umsetzung betrug 71 Prozent, die Selektivität zu Dimethylether 56 Prozent und zu Methanol 19 Prozent. Der Anteil des Methanolkatalysators in der Schüttung lag bei 47,5 Gewichtsprozent.

Wie aus Figur 2 ersichtlich, ergibt sich in dieser nicht erfindungsgemäßen Ausgestaltung ein ausgesprochen hoher Temperaturpeak am Anfang der Schüttung.

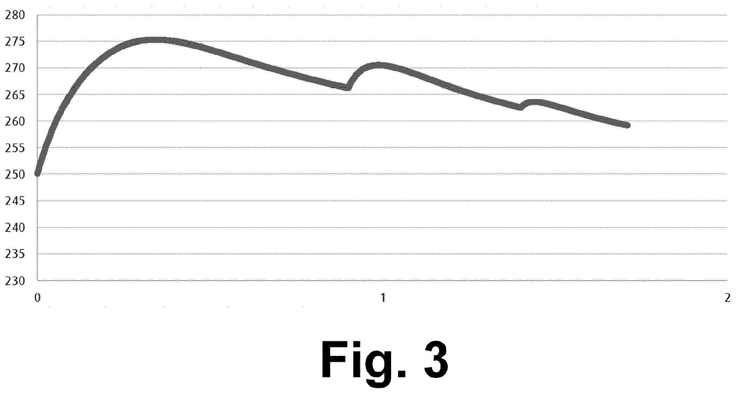

Figur 3 zeigt ein Temperaturprofil bei Verwendung eines Synthesereaktors bzw. Verfahrens gemäß einem nicht erfindungsgemäßen Vergleichsbeispiel. In dem nicht erfindungsgemäßen Vergleichsbeispiel wurde eine Temperaturkontrolle durch Variation der Katalysatormischung (aufsteigender Anteil an Methanolkatalysator und sinkender Anteil des Dimethyletherkatalysatoranteils in der Schüttung) mit fester Aktivität des Methanolkatalysators vorgenommen, und zwar unter gleichen Bedingungen wie zu Figur 2 erläutert. Die Temperaturkontrolle gemäß diesem nicht erfindungsgemäßen Vergleichsbeispiel geht auf Kosten der Konversion und des Zielprodukts Dimethylether. Die Konversion betrug hier nur 69 Prozent, die Selektivität zu Dimethylether sank auf 51 Prozent, während sich die Selektivität zu Methanol auf 25 Prozent erhöhte.

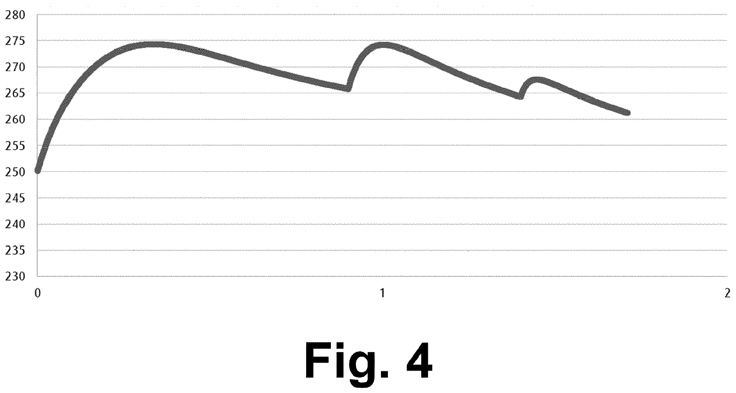

Figur 4 zeigt ein Temperaturprofil bei Verwendung eines Synthesereaktors bzw. Verfahrens gemäß einer Ausgestaltung der Erfindung. Bei der hier vorgenommenen Temperaturkontrolle über die Katalysatoraktivität, anstatt nur die Katalysatormischung, gelingt eine Temperaturkontrolle und darüber hinaus kann sogar die Konversion gesteigert werden, da bei niedrigeren Temperaturen höhere Katalysatoraktivitäten des Methanolkatalysators vorliegen. Die Konversion betrug hier 74 Prozent, die Selektivität zu Dimethylether 55 Prozent und die Selektivität zu Methanol 21 Prozent. Die Anteile des Methanol- und Dimethyletherkatalysators wurden in den Teilzonen gleich gehalten, wobei die Katalysatoraktivität des Methanolkatalysators je Teilzone jedoch aufsteigend gewählt wurde.

## Patentansprüche

1. Verfahren (100) zur Herstellung von Dimethylether, bei dem ein die Reaktanden Kohlenmonoxid und/oder Kohlendioxid und Wasserstoff enthaltendes Synthesegas in einer Strömungsrichtung unter teilweiser Umsetzung der Reaktanden und Bildung von Methanol an einem oder mehreren Methanolkatalysatoren und Umsetzung zumindest eines Teils des Methanols an einem oder mehreren Dimethyletherkatalysatoren durch einen Synthesereaktor (10) geführt wird, in dem der Methanolkatalysator und der Dimethyletherkatalysator in einer gemeinsamen Katalysezone (14) bereitgestellt sind, **dadurch gekennzeichnet, dass** die Katalysezone (14) zumindest zwei Teilzonen (A, B, C) aufweist, die in Strömungsrichtung hintereinander angeordnet sind, wobei die Teilzonen (A, B, C) den oder die Methanolkatalysatoren in ansteigenden Aktivitäten aufweisen.

2. Verfahren (100) nach Anspruch 1, bei dem der Methanolkatalysator oder zumindest einer der mehreren Methanolkatalysatoren Kupfer aufweist und/oder der Dimethyletherkatalysator oder zumindest einer der mehreren Dimethyletherkatalysatoren eine saure Komponente aufweist.

3. Verfahren (100) nach Anspruch 2, bei dem der Methanolkatalysator oder zumindest einer der mehreren Methanolkatalysatoren die Komponenten Kupfer, Zinkoxid und Dialuminiumtrioxid aufweist und/oder der Dimethyletherkatalysator oder zumindest einer der mehreren Dimethyletherkatalysatoren ein oder mehrere Gamma-Aluminiumoxide, Sirale und/oder Zeolithe aufweist.

4. Verfahren (100) nach einem der vorstehenden Ansprüche, bei dem in den Teilzonen (A, B, C) mehrere unterschiedliche Methanolkatalysatoren und/oder mehrere unterschiedliche Dimethyletherkatalysatoren mit unterschiedlicher Wasser- und/oder Kohlendioxidverträglichkeit verwendet werden.

5. Verfahren (100) nach einem der vorstehenden Ansprüche, bei dem in den Teilzonen (A, B, C) jeweils eine physikalische Mischung des einen oder der mehreren Methanolkatalysatoren und des einen oder der mehreren Dimethyletherkatalysatoren eingebracht sind.

6. Verfahren (100) nach Anspruch 5, bei dem die physikalische Mischung jeweils 40 bis 80 oder 50 bis 75 Gewichtsanteile des einen oder der mehreren Methanolkatalysatoren und in einem verbleibenden Gewichtsanteil den einen oder der mehreren Dimethyletherkatalysatoren aufweist.

7. Verfahren (100) nach einem der vorstehenden Ansprüche, bei dem der Synthesereaktor (10) ein Rohrreaktor ist, in dem Reaktionsrohre (12) angeordnet sind, die zumindest zum Teil von einem Kühlmedium umströmt werden.

8. Verfahren (100) nach Anspruch 7, bei dem das Kühlmedium aus einem oder mehreren Thermoölen, Siedewasser und einer oder mehreren Salzschmelzen oder Kombinationen hiervon ausgewählt ist oder sind.

9. Verfahren (100) nach Anspruch 7 oder 8, bei dem eine Temperatur des Kühlmediums in einem Bereich von 200 bis 300 oder 230 bis 280 °C liegt.

10. Verfahren (100) nach einem der vorstehenden Ansprüche 7 bis 9, bei dem zumindest ein Teil der Reaktionsrohre (12) einen Innendurchmesser von 20 bis 60 oder 24 bis 50 mm aufweist.

11. Verfahren (100) nach einem der vorstehenden Ansprüche 7 bis 10, bei dem ein gesamtes katalytisches Volumen, das innerhalb der Reaktionsrohre (12) angeordnet ist, mit einer stündlichen Gasraumgeschwindigkeit von 1.500 bis 5.000 oder 2.000 bis 4.000 pro Stunde durchströmt wird.

12. Verfahren (100) nach einem der vorstehenden Ansprüche, bei das Synthesegas auf einer Temperatur in einem Bereich von 200 bis 300, 230 bis 280 oder 240 bis 275 °C durch den Synthesereaktor geführt wird.

13. Anlage zur Herstellung von Dimethylether, die einen Synthesereaktor (10) aufweist, wobei die Anlage Mittel aufweist, die dafür eingerichtet sind, ein die Reaktanden Kohlenmonoxid und/oder Kohlendioxid und Wasserstoff enthaltendes Synthesegas in einer Strömungsrichtung unter teilweiser Umsetzung der Reaktanden und Bildung von Methanol an einem oder mehreren Methanolkatalysatoren und Umsetzung zumindest eines Teils des Methanols an einem oder mehreren Dimethyletherkatalysatoren durch den Synthesereaktor (10) zu führen, wobei in dem Synthesereaktor (10) der Methanolkatalysator und der Dimethyletherkatalysator in einer gemeinsamen Katalysezone (14) bereitgestellt sind, **dadurch gekennzeichnet, dass** die Katalysezone (14) zumindest zwei Teilzonen (A, B, C) aufweist, die in der Strömungsrichtung hintereinander angeordnet sind, wobei die Teilzonen (A, B, C) den oder die Methanolkatalysatoren in ansteigenden Aktivitäten aufweisen.

14. Anlage nach Anspruch 13, die zur Durchführung eines Verfahrens nach einem der vorstehenden Ansprüche eingerichtet ist.
